# EUROPEAN PATENT APPLICATION

(11) **EP 2 910 174 A1**
(43) Date of publication of application: **26.08.2015**
(21) Application number: 13847721.1
(22) Date of filing: 27.09.2013
(51) Int. Cl.: A61B 1/00, G02B 23/26

(54) **OBSERVATION DEVICE, OBSERVATION ASSISTANCE DEVICE, OBSERVATION ASSISTANCE METHOD AND PROGRAM**

(30) Priority: 16.10.2012 JP 2012229254
(71) Applicant: Olympus Corporation, Shibuya-ku Tokyo 151-0072 (JP)
(72) Inventor: FUJITA, Hiromasa, Shibuya-ku Tokyo 151-0072 (JP); TOJO, Ryo, Hachioji-shi Tokyo 192-8512 (JP)
(74) Representative: Winter, Brandl, Fürniss, Hübner, Röss, Kaiser, Polte - Partnerschaft mbB
(86) International application number: PCT/JP2013/076398
(87) International publication number: WO 2014/061428

(57) **Abstract**

An inserting state acquiring section (51) acquires inserting state information of an inserting section that is to be inserted into an insertion subject, and an insertion subject shape acquiring section (52) acquires shape information of the insertion subject. A positional relation calculating section (54) is input the inserting state information and the insertion subject shape information and calculates a positional relation of the inserting section to the insertion subject, and an output section (55) outputs the calculation result of the positional relation calculating section (54) as display information.

## Description

### Technical Field

The present invention relates to an observation apparatus in which an inserting section is inserted into an insertion subject for observation, an observation supporting device for use in such an observation apparatus, an observation supporting method, and a program which allows a computer to execute a procedure of the observation supporting device.

### Background Art

As a supporting device in a case where an inserting section is inserted into an insertion subject for observation, for example, there is disclosed, in U.S. Patent No. 6,846,286, a constitution to display a shape of an endoscope inserting section in a display section when the endoscope inserting section is inserted into a human body.

As to this constitution, in an endoscope device, flexible bend detecting optical fibers having bend detecting portions in which a quantity of light to be transmitted changes in accordance with a size of an angle of a bend are attached to a flexible band-like member in a state where the fibers are arranged in parallel, and the band-like member is inserted into and disposed in the endoscope inserting section along a substantially total length of the endoscope inserting section. Additionally, a bending state of the band-like member in a portion where each bend detecting portion is positioned is detected from the light transmission quantity of each bend detecting optical fiber, to display the bending state as the bending state of the endoscope inserting section in a monitor screen.

In general, there are only a few regions that become marks in an insertion subject, and hence when it is not easily judged only from an acquired image which region of the insertion subject is being observed, it is also not easily judged whether or not all required regions could be imaged (observed).

In a technology disclosed in U.S. Patent No. 6,846,286 mentioned above, it is possible to display a shape of an inserting section in the insertion subject which cannot be seen from the outside of the insertion subject when the inserting section is inserted into the insertion subject. However, there has not been suggested a method of detecting and displaying which region of the insertion subject is being imaged (observed).

### Summary of Invention

The present invention has been developed in respect of the above, and an object thereof is to provide an observation apparatus, an observation supporting device, an observation supporting method and a program that can supply, to an operator, information to judge which region of an insertion subject is being imaged.

According to a first aspect of the invention, there is provided an observation apparatus characterized by comprising: an inserting section that is to be inserted into an insertion subject; an image acquisition section that is disposed in the inserting section to acquire image of the insertion subject; an inserting state acquiring section that acquires inserting state information of the inserting section; an insertion subject shape acquiring section that acquires shape information of the insertion subject; a positional relation calculating section that is input the inserting state information and the insertion subject shape information and calculates a positional relation of the inserting section to the insertion subject; and an output section that outputs the calculation result of the positional relation calculating section as display information.

According to a second aspect of the invention, there is provided an observation supporting device for use in an observation apparatus in which an inserting section is inserted into an insertion subject to acquire image of the inside of the insertion subject, the observation supporting device comprising: an inserting state acquiring section that acquires inserting state information of the inserting section; an insertion subject shape acquiring section that acquires shape information of the insertion subject; a positional relation calculating section that is input the inserting state information and the insertion subject shape information and calculates a positional relation of the inserting section to the insertion subject; and an output section that outputs the calculation result of the positional relation calculating section as display information.

According to a third aspect of the invention, there is provided an observation supporting method for use in an observation apparatus in which an inserting section is inserted into an insertion subject to acquire image of the inside of the insertion subject, the method comprising: an inserting state acquiring step of acquiring inserting state information of the inserting section; an insertion subject shape acquiring step of acquiring shape information of the insertion subject; a positional relation calculating step of being input the inserting state information and the insertion subject shape information, and calculating a positional relation of the inserting section to the insertion subject; and an output step of outputting the calculation result of the positional relation calculating step as display information.

According to a forth aspect of the invention, there is provided a program which allows a computer to execute: an inserting state acquiring procedure of acquiring inserting state information of an inserting section in an observation apparatus in which the inserting section is inserted into an insertion subject to acquires image of the inside of the insertion subject; an insertion subject shape acquiring procedure of acquiring shape information of the insertion subject; a positional relation calculating procedure of being input the inserting state information and the insertion subject shape information, and calculating a positional relation of the inserting section to the insertion subject; and an output procedure of outputting the calculation result of the positional relation calculating procedure as display information.

According to the present invention, it is possible to supply information to judge which region of an insertion subject is being imaged, and hence an operator can easily judge which region of the insertion subject is being imaged and whether or not all required regions could be imaged. Therefore, it is possible to provide an observation apparatus, an observation supporting device, an observation supporting method and a program which can prevent oversight of observation regions.

### Brief Description of Drawings

FIG. 1A is a view showing a schematic constitution of an observation apparatus to which an observation supporting device according to a first embodiment of the present invention is applied;
FIG. 1B is a block diagram of the observation supporting device according to the first embodiment;
FIG. 1C is a view for explaining an example where information is supplied via a display device connected to the observation supporting device according to the first embodiment;
FIG. 2A is a view showing a schematic constitution of a hard endoscope device as an inserting tool in the observation apparatus according to the first embodiment;
FIG. 2B is a perspective view of a distal end of an inserting section;
FIG. 3A is a view for explaining a constitution of an insertion and rotation detecting section;
FIG. 3B is a view for explaining an operation principle of the insertion and rotation detecting section;
FIG. 4 is a view showing an inserting state of the inserting section into an insertion subject;
FIG. 5 shows an operation flowchart of the observation supporting device according to the first embodiment;
FIG. 6A is a view for explaining which position of the insertion subject is to be displayed by a first position display;
FIG. 6B is a view for explaining which position of the insertion subject is to be displayed by a second position display;
FIG. 7A is a view for explaining a display example where the inserting section is inserted into a branched insertion subject;
FIG. 7B is a view for explaining another display example;
FIG. 7C is a view for explaining still another display example;
FIG. 8A is a view showing a state before rotation to explain a change of an acquired image due to the rotation of the inserting section;
FIG. 8B is a view showing a state after the rotation to explain the change of the acquired image due to the rotation of the inserting section;
FIG. 9 is a view showing a schematic constitution of a soft endoscope device as an inserting tool in an observation apparatus according to a second embodiment of the present invention;
FIG. 10 is a view showing an inserting state of the inserting section into an insertion subject;
FIG. 11 is a view showing a schematic constitution of the observation apparatus according to the second embodiment;
FIG. 12A is a view showing a case where a bending portion is bent in an upward direction of the paper surface to explain a principle of a fiber shape sensor;
FIG. 12B is a view showing a case where the bending portion is not bent to explain the principle of the fiber shape sensor:
FIG. 12C is a view showing a case where the bending portion is bent in a downward direction of the paper surface to explain the principle of the fiber shape sensor;
FIG. 13 is a view showing an attaching structure of the fiber shape sensor to the inserting section;
FIG. 14 is a block diagram of an observation supporting device according to a third embodiment;
FIG. 15 is a view showing an operation flowchart of the observation supporting device according to the third embodiment;
FIG. 16 is a view for explaining an example where information is supplied in an observation apparatus according to the third embodiment;
FIG. 17 is a view for explaining another example of an information supply configuration; and
FIG. 18 is a view for explaining still another example of the information supply configuration.

### Description of Embodiments

Hereinafter, a mode for carrying out the present invention will be described with reference to the drawings.

### [First Embodiment]

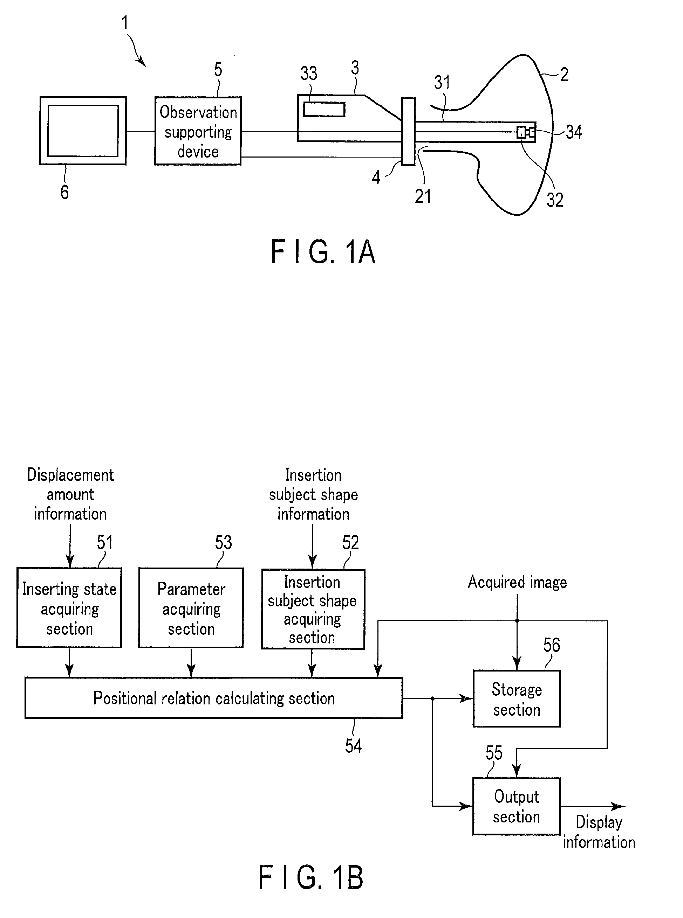

As shown in FIG. 1A, an observation apparatus 1 concerned with a first embodiment of the present invention is constituted of an inserting tool 3 including an inserting section 31 to be inserted into an insertion subject 2 and an image acquisition section 32 that acquires image of the insertion subject 2; an insertion and rotation detecting section 4 as a detecting section that detects displacement amount information of the inserting section 31; an observation supporting device 5 concerned with the first embodiment of the present invention which acquires inserting state information of the inserting section 31 from the displacement amount information from the insertion and rotation detecting section 4 and calculates a positional relation of the inserting section 31 and the insertion subject 2 on the basis of the inserting state information and shape information of the insertion subject 2 to output the calculation result as display information; and a display device 6 that displays the display information.

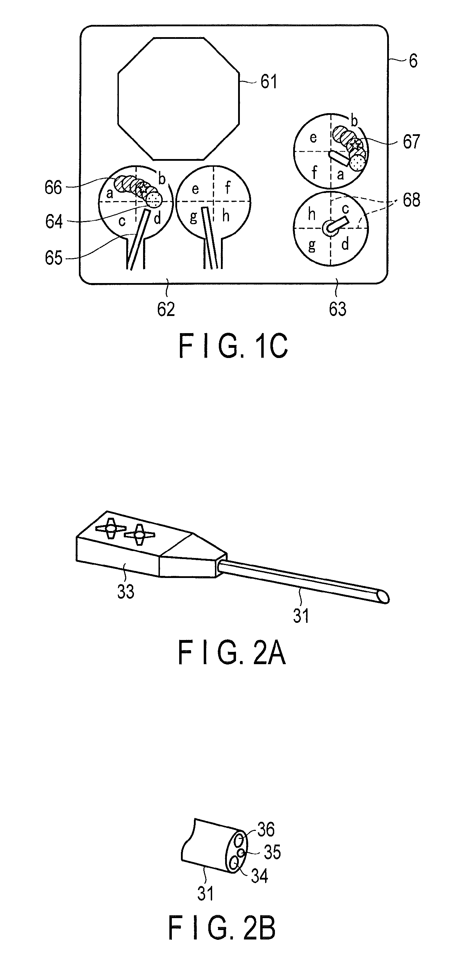

The inserting tool 3 is, for example, such a hard endoscope device as shown in FIG. 2A and includes the inserting section 31 and an operating section 33 constituted integrally with the inserting section 31. The inserting section 31 is a hard tubular member and is insertable from an insertion port 21 of the insertion subject 2 into the insertion subject 2. It is to be noted that the insertion subject 2 is filled with a predetermined material such as air, physiological saline or a chemical solution. In an end portion of the inserting section 31 in an inserting direction (hereinafter referred to as an inserting section distal end), as shown in FIG. 2B, an image acquisition opening 34 is disposed, and in the vicinity of the inserting section distal end in the inserting section 31, as shown in FIG. 1A, the image acquisition section 32 is included. Light entering into the image acquisition opening 34 is received by the image acquisition section 32 that performs image acquisition. An image acquired by the image acquisition section 32 is output to the display device 6 through the observation supporting device 5 concerned with the present first embodiment.

It is to be noted that needless to say, the image acquisition section 32 may not be disposed in the vicinity of the inserting section distal end of the inserting section 31 but may be disposed in the operating section 33 and connected to the image acquisition opening 34 by a light guide or the like to guide the light entering into the image acquisition opening 34 to the image acquisition section 32 that performs the image acquisition.

In addition, although not especially shown in the drawing, the inserting section 31 has an illuminating optical fiber therein, and light from an unshown illuminating light source disposed in the operating section 33 is guided to exit as illumination light for the image acquisition from a light supplying portion 35 at the inserting section distal end. Further, at the inserting section distal end, a treating opening 36 is disposed, and a treatment tool inserted from the operating section 33 into the inserting section 31 can extend from the treating opening 36 to the outside of the inserting section 31.

Hereinafter, a constitution of each section will be described in detail.

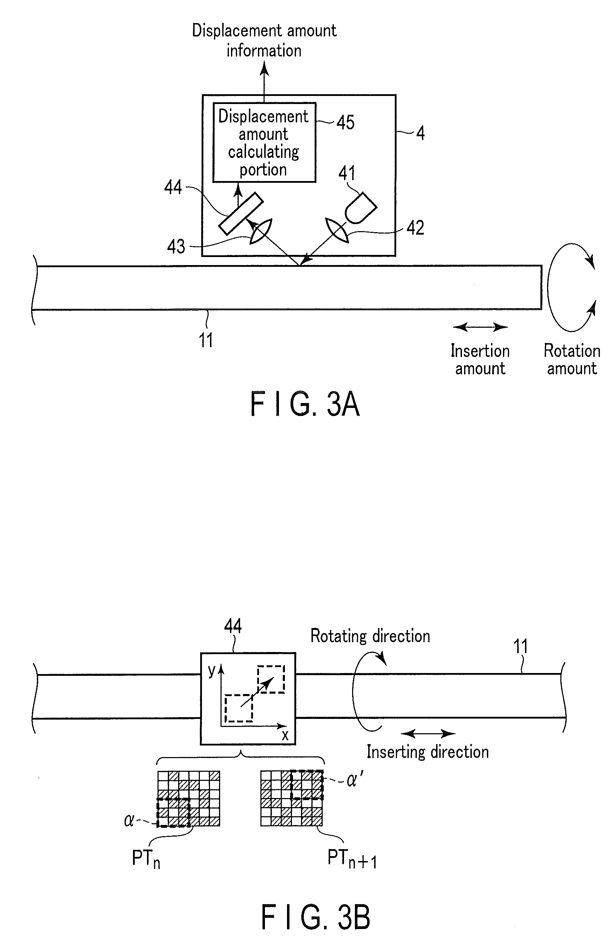

In addition, the insertion and rotation detecting section 4 is disposed in the vicinity of the insertion port 21 of the insertion subject 2, and detects an insertion amount and a rotation amount of the inserting section 31 to output the amounts as one piece of the displacement amount information of the inserting section 31 to the observation supporting device 5. Specifically, as shown in FIG. 3A, the insertion and rotation detecting section 4 is constituted of a light source 41, a projection lens 42, a light receiving lens 43, an optical pattern detecting portion 44, and a displacement amount calculating portion 45.

The inserting section 31 is irradiated with the light emitted from the light source 41 through the projection lens 42, and the light reflected by the inserting section 31 is received through the light receiving lens 43 by the optical pattern detecting portion 44. The optical pattern detecting portion 44 detects images of a plane of the inserting section 31 which is an optical pattern continuously at detection times t₀, t₁, t₂, ..., tₙ, ....

As shown in FIG. 3B, the displacement amount calculating portion 45 compares a displacement in image data of any selected reference pattern α that is present in the image (an optical pattern PTₙ) of the image data acquired at any time tₙ by the optical pattern detecting portion 44 with a displacement in image data of an optical pattern α' that is present in a part of an image (an optical pattern PTₙ₊₁) of the image data acquired at any time tₙ₊₁ after the elapse of time from the above time tₙ and that matches the above reference pattern α, and the displacement amount calculating portion calculates a displacement amount in each image in an x-axis direction and a y-axis direction. Here, as shown in FIG. 3B, the optical pattern detecting portion 44 is positioned so that an x-axis of the optical pattern detecting portion 44 matches an axial direction of the inserting section 31. Therefore, a displacement amount Δx_{f} in the x-axis direction which is calculated by the displacement amount calculating portion 45 is proportional to the insertion amount of the inserting section 31, and a displacement amount Δy_{f} in the y-axis direction is proportional to the rotation amount of the inserting section 31. The displacement amounts (the insertion amount and the rotation amount) in the images which are calculated by the displacement amount calculating portion 45 are output as the displacement amount information to the observation supporting device 5. It is to be noted that an increase/decrease direction of each displacement amount indicates directions of insertion and rotation of the inserting section 31, and hence the displacement amount information also includes information of the inserting direction and the rotating direction.

Additionally, as shown in FIG. 1B, the observation supporting device 5 concerned with the present embodiment is constituted of an inserting state acquiring section 51, an insertion subject shape acquiring section 52, a parameter acquiring section 53, a positional relation calculating section 54, an output section 55, and a storage section 56.

The inserting state acquiring section 51 acquires inserting state information of at least a part of the inserting section 31 inserted into the insertion subject 2, e.g., a position and a direction of a certain point of the inserting section 31 on the basis of the displacement amount information output from the displacement amount calculating portion 45 of the insertion and rotation detecting section 4.

The insertion subject shape acquiring section 52 acquires the shape information of the insertion subject 2 (the insertion subject shape information). This insertion subject shape information is constituted on the basis of data from the outside or inside of the insertion subject 2 before the inserting section 31 is inserted into the insertion subject 2.

That is, the insertion subject shape information based on the data from the outside is constituted by utilizing an apparatus that can detect the information by use of the light transmitted through the insertion subject 2, for example, a CT diagnosis apparatus, an ultrasonic diagnosis apparatus or an X-ray apparatus.

In addition, the insertion subject shape information based on the data from the inside is constituted by utilizing locus data obtained when the inserting section 31 is moved in a space of the insertion subject 2 or by connecting position information obtained when the inserting section distal end comes in contact with the insertion subject 2. When the position information obtained during the contact between the inserting section distal end and the insertion subject 2 is utilized, a size of the space can be detected, and the insertion subject shape information can more exactly be acquired. Furthermore, when the insertion subject 2 is a human organ, the information may be constituted by presuming a physical constitution, and when the insertion subject 2 is a structure, the information may be constituted by inputting the shape through a drawing.

It is to be noted that when the insertion subject shape information is acquired by the insertion subject shape acquiring section 52, the insertion subject shape information may directly be acquired from an apparatus such as the CT diagnosis apparatus by connecting the apparatus that constitutes the insertion subject shape information, or the insertion subject shape information may be acquired by storing the insertion subject shape information output from the apparatus once in a storage medium and reading the stored insertion subject shape information or by downloading the insertion subject shape information via a network. Furthermore, the insertion subject shape acquiring section 52 is not limited to that interface or data reader and the acquiring section itself may be the apparatus that constitutes the insertion subject shape information.

The parameter acquiring section 53 acquires parameters to be utilized in calculation of the positional relation calculating section 54, for example, view angle information of the image acquisition section 32 (a focal length of the lens or the like) and a refractive index of a predetermined material such as air or the chemical solution interposed between the inserting section distal end and the insertion subject 2. The parameter acquiring section 53 may input the parameters from the outside to acquire the parameters or may store the parameters in advance.

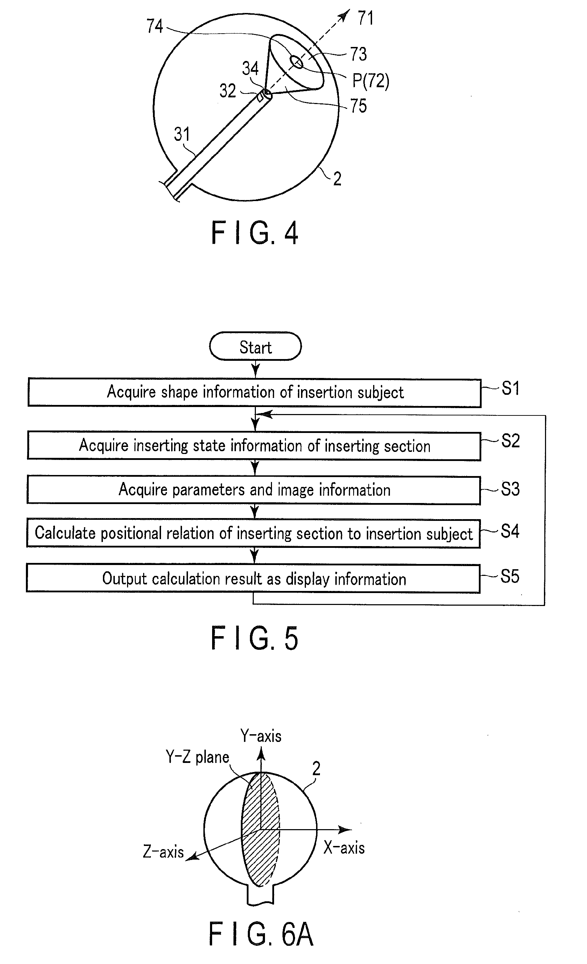

The positional relation calculating section 54 calculates a positional relation of the inserting section 31 to the insertion subject 2, i.e., a specific position of the insertion subject 2 to which the whole inserting section 31 or the distal end of the inserting section is directed, on the basis of already known shape information of the inserting section 31 which is stored beforehand, the inserting state information acquired by the inserting state acquiring section 51, the insertion subject shape information acquired by the insertion subject shape acquiring section 52, the parameters acquired by the parameter acquiring section 53, and the image acquired by the image acquisition section 32 (the acquired image). Specifically, the positional relation calculating section 54 first calculates a position of the inserting section distal end on the basis of the shape information and inserting state information of the inserting section 31, and calculates a movement amount and a moving direction of the inserting section distal end, i.e., a direction (an axial direction) in which the inserting section distal end is directed, from the movement of the optical pattern in the acquired image in the same manner as in the insertion and rotation detecting section 4. During this calculation, a correction is added in accordance with the parameter acquired by the parameters acquiring section 53. Furthermore, an intersection between the direction in which the inserting section distal end is directed and the insertion subject 2 is calculated on the basis of the calculation results and the insertion subject shape information. That is, as shown in FIG. 4, the positional relation calculating section 58 obtains, as an image acquisition position P, an intersection 72 between a straight line including a direction in which the inserting section distal end is directed (an image acquisition direction 71) and the shape of the insertion subject 2, i.e., the center of a viewing field (an image acquisition region 73).

In general, a region of interest in an observation object is at the center of the viewing field, and hence the center of the viewing field is often more important than a periphery thereof. It is to be noted that here the description has been given as to the example where the intersection is obtained as the image acquisition position P, but the viewing field (the image acquisition region 73) that is a region of the insertion subject 2 imaged by the image acquisition section 32 may be calculated as the image acquisition position P from a distance between the position of the inserting section distal end and an image acquisition plane of the insertion subject 2 on the basis of the insertion subject shape information. In this case, the image acquisition region 73 can more exactly be obtained by using parameters such as the refractive index of the predetermined material interposed between the inserting section 31 and the insertion subject 2 or the view angle information of the image acquisition section 32 (the focal length of the lens or the like). The image acquisition region 73 is obtained as the image acquisition position P in this manner, so that a region imaged by the image acquisition section 32 can be grasped. In addition, a partial region 74 or a point in the viewing field (the image acquisition region 73) may be calculated as the image acquisition position P. For example, when the image acquisition region 73 cannot exactly be detected, a small region is calculated in consideration of an error, so that a region that is not imaged can be prevented from being wrongly detected as the imaged region. That is, an omission of observation can be prevented.

The positional relation calculating section 54 outputs image acquisition position information indicating the image acquisition position P obtained as described above to the output section 55 and the storage section 56.

The output section 55 prepares the display information to display the calculation result of the positional relation calculating section 54, i.e., the image acquisition position information indicating the above image acquisition position P (e.g., the intersection 72) by the display device 6 in a configuration where an operator can judge a specific position of the insertion subject 2 to which the inserting section distal end is directed, and the output section outputs the display information to the display device 6.

The storage section 56 stores at least a part of the calculation result of the positional relation calculating section 54 and stores the acquired image as required.

An operation of the observation supporting device 5 having such a constitution as described above will be described with reference to FIG. 5.

First, the insertion subject shape acquiring section 52 acquires the insertion subject shape information (step S1). Afterward, the inserting state acquiring section 51 acquires the inserting state information of the inserting section 31 into the insertion subject 2 (step S2). Furthermore, the positional relation calculating section 54 acquires the parameters from the parameter acquiring section 53 and the acquired image from the image acquisition section 32 (step S3), and calculates the positional relation of the inserting section 31 to the insertion subject 2 on the basis of the insertion subject shape information, the inserting state information, the parameters and the acquired image (step S4). Afterward, the calculation result is stored in the storage section 56 and is also output by the output section 55 (step S5). The processing then returns to the step S2 to repeat the above operation of the step S2 to the step S5.

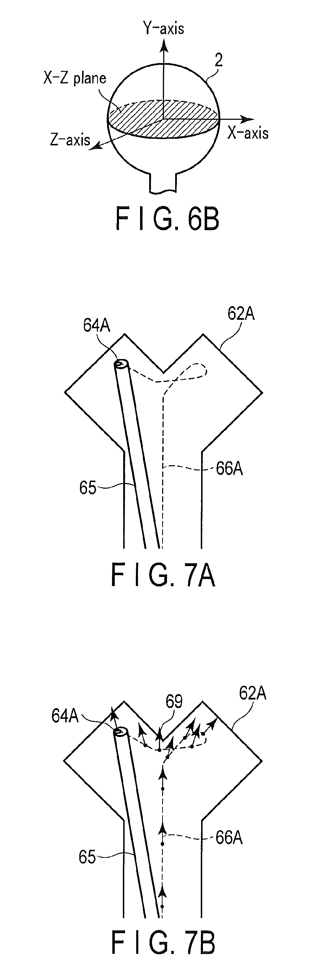

According to this operation, such a display as shown in FIG. 1C is made by the display device 6 connected to the observation supporting device 5. That is, in the present embodiment, the output section 55 prepares such display information as to display, by the display device 6, the acquired image from the image acquisition section 32 (an acquired image display 61) and two-dimensional views 62 and 63 as the insertion subject shape information in which the insertion subject 2 is divided by a predetermined region, to output the display information. Furthermore, the output section prepares such display information as to display information concerning the image acquisition position P, i.e., the intersection 72 between the insertion subject 2 and the axial direction (the image acquisition direction 71) of the inserting section distal end further on the two-dimensional views 62 and 63 in which the insertion subject 2 is divided by the predetermined region. Here, the first two-dimensional view 62 is a view showing a state where the shape of the insertion subject 2 is divided by a Y-Z plane and opened in a right-left direction at a coordinate of the insertion subject 2 as shown in FIG. 6A, and the second two-dimensional view 63 is a view that shows, as a view having a view point different from that of the first two-dimensional view 62, a state where the shape of the insertion subject 2 is divided by an X-Z plane and opened in an upward-downward direction at the coordinate of the insertion subject 2 as shown in FIG. 6B. Furthermore, the output section 55 prepares such display information as to display a current position display 64 as the information on the image acquisition position P on the two-dimensional views 62 and 63.

It is to be noted that the current position display 64 may be the intersection 72 itself between the insertion subject 2 and the axial direction of the inserting section distal end, but is preferably the image acquisition region 73 as described above or the region 74 of a part in the image acquisition region around, for example, the intersection 72, because the region having a certain degree of range in this manner is more easily visible.

In addition, the output section 55 can prepare such display information as to display an inserting section shape schematic display 65 showing the shape of the inserting section 31 in addition to the current position display 64 as the information concerning the current image acquisition position P. That is, the position of the inserting section distal end is calculated by the positional relation calculating section 54 as described above and the shape information of the inserting section 31 is already known, and hence it is possible to know the inserting state of the inserting section 31 inserted into the insertion subject 2 and perform the inserting section shape schematic display 65.

Furthermore, the output section 55 can prepare such display information as to display a position locus display 66 by use of the calculation result stored in the storage section 56. The position locus display 66 also has a certain degree of range in the same manner as in the current position display 64. Additionally, in this case, such display information as to achieve some identification display is preferably prepared by changing mutual colors, concentrations or patterns or by performing the position locus display 66 as a blinking display so that the current position display 64 and the position locus display 66 can be distinguished. The operator may be allowed to select the presence/absence of this identification display or a configuration of the identification display.

In addition, there may be added a function that the operator can make a marking 67 in, for example, an already observed region or a region required to be observed hereafter or again. For example, in response to a predetermined button operation of the operating section 12, the information of the corresponding region is stored in the storage section 56, and the output section 55 also changes the display information so as to display the marking 67 in the region. This region of the marking 67 may be fixed to one of the intersection 72, the image acquisition region 73, and the region 74 of a part in the image acquisition region, or may arbitrarily be set by the operator. The marking 67 is enabled in this manner, thereby enabling confirmation of the observed region or the region required to be observed again in the insertion subject 2, a region that requires some treatment (removal, sampling, repair, or the like), or the like. Furthermore, it is possible to utilize the marking when the previous region required to be observed again is specified or the inserting section distal end is allowed to quickly reach the corresponding region in a case where the insertion subject 2 is observed again at a different opportunity. In addition, during the storage, when not only the information of the region provided with the marking 67 but also the inserting direction of the inserting section 31 and the like are stored, the marking can be utilized in a case where the confirmation is performed after the observation is performed, a case where the observation is next performed in the same state, or the like. It is to be noted that when information to specify each of the observed region, the region required to be observed again and the region required to be treated can also be set and stored, the operator can accordingly change a color or shape of the configuration of the display to easily judge a meaning of each of the markings 67.

In addition, concerning the marking 67, the observation supporting device 5 may be connected to a pointing device or a visual recognition device to allow the operator to designate any range or point on the acquired image display 61 or the two-dimensional view 62 or 63 displayed by the display device 6.

Additionally, in the two-dimensional views 62 and 63 shown in FIG. 1C, a region dividing display 68 is performed. In the views, there are displayed two or more regions which are divided in accordance with a common theory or stipulation of a learned society or for standard utilization, or divided by a predetermined dividing method. In this case, it becomes easy for the operator to identify certain positions in the insertion subject 2.

It is to be noted that the insertion and rotation detecting section 4 optically detects the shape of the inserting section 31 inserted into the insertion subject 2 and a position and a direction of the image acquisition opening 34 as described above, but may detect the same by another method. For example, a coil is disposed in the vicinity of at least the image acquisition opening 34 in the inserting section 31 and a current is passed through the coil to generate a magnetic field which is received on the outside, or a magnetic field distribution generated on the outside is received by the coil, so that the position or direction of the coil, i.e., the image acquisition opening 34 can be detected.

As described above, according to the present embodiment, the inserting state acquiring section 51 acquires the inserting state information (e.g., the position or direction of the certain point of the inserting section 31 (the inserting section distal end)) of (at least a part of) the inserting section 31 (inserted into the insertion subject 2) which is to be inserted into the insertion subject 2, and the insertion subject shape acquiring section 52 acquires the shape information of the insertion subject 2. Furthermore, the inserting state information and the insertion subject shape information are input into the positional relation calculating section 54 to calculate the positional relation of the inserting section 31 to the insertion subject 2 (the position or direction of the whole inserting section 31 or the inserting section distal end), and the output section 55 outputs the calculation result of the positional relation calculating section 54 as the display information, so that it is possible to supply information to judge which region of the insertion subject is being imaged. That is, when the inside of the insertion subject which cannot be seen directly by eye is observed with the inserting tool 3 having the inserting section 31, it is possible to approximate which place or which direction of the insertion subject 2 is being observed in the acquired image displayed by the display device 6. In addition, the observed region or an unobserved region in the insertion subject 2 can easily be identified, and oversight can be prevented.

In addition, the positional relation calculating section 54 obtains the positional relation of the inserting section 31 to the insertion subject 2 from a distance between the position of the inserting section distal end and the image acquisition plane of the insertion subject 2, and the output section 55 outputs this positional relation as the display information, so that an observation range of the insertion subject 2 (the positional relation of the inserting section 31 to the insertion subject 2) can be displayed by the display device 6. In consequence, when the inside of the insertion subject 2 is observed with the inserting section distal end, the inserting section distal end can quickly be moved to the region to be observed next. Furthermore, the inserting section 31 can be moved without any oversight.

In addition, when a predetermined material such as the physiological saline or chemical solution is present between the insertion subject 2 and the inserting section 31, refraction of light is generated due to a difference in refractive index, and the observation range of the insertion subject 2 by the image acquisition section 32 (the positional relation of the inserting section 31 to the insertion subject 2) varies. However, the positional relation calculating section 54 further inputs the refractive index of the predetermined material interposed between the inserting section 31 and the insertion subject 2 from the parameter acquiring section 53 to obtain the positional relation of the inserting section 31 to the insertion subject 2, so that the observation range can more exactly be obtained.

Furthermore, an image acquisition range 75 (see FIG. 4) of the image acquisition section 32 also varies with view angle information of the image acquisition section 32 (the focal length of the lens or the like), and hence the positional relation calculating section 54 inputs the view angle information of the image acquisition section 32 from the parameter acquiring section 53 to obtain the positional relation of the inserting section 31 to the insertion subject 2, so that the observation range can more exactly be obtained.

In addition, the observation supporting device further has the storage section 56 that stores at least a part of the calculation result of the positional relation calculating section 54, and the storage section 56 stores the observation ranges of the insertion subject 2 which are displayed in the display device 6 (the positional relations of the inserting section 31 to the insertion subject 2), so that it is possible to identify the regions observed with the inserting section distal end and the unobserved region to an inner wall of the insertion subject 2. For example, when the observation range displayed as the current position display 64 by the display device 6 is left as the position locus display 66 without being erased in a case where the inserting section distal end is moved to another region, it is possible to easily judge the observed region and the unobserved region by the position locus display 66. This constitution enables the observation of the whole insertion subject 2 without any oversight.

In addition, the output section 55 outputs the display information to display two or more different predetermined states by a distinguishable method, for example, to display the markings that can be distinguished as the result of the positional relation calculating section 54, so that it is possible to apparently distinguish the states (the current observation range, cancer, inflammation, defect, wound, corrosion, etc.). When these states are stored beforehand, solely the region that is in the predetermined state can be observed in a case where the same place is observed again at a different time, so that observation efficiency can improve.

It is to be noted that classification of the predetermined states can be selected by the operator of the inserting section 31. In this case, the predetermined states can be classified on the basis of the operator's intention, and hence a standard of re-observation or the like can arbitrarily be set by the operator or a standard of a field to which the operator belongs. That is, it is possible to use the device appropriately, in accordance with the field, such as a medical field and an industrial field.

In addition, the storage section 56 stores the acquired image, with the image being associated with the calculation result of the positional relation calculating section 54, so that as to the state of the predetermined region, an observation state when the state was stored can be compared with the current observation state to obtain a difference between the states or a change of the state.

In addition, the output section 55 outputs the display information in which the region dividing displays are carried out in the regions. In consequence, when the region dividing displays are carried out in a display screen in accordance with the common theory or stipulation of the learned society or so that the region for standard utilization can be seen, it is possible to easily understand which position is being observed in a case where the observation range is displayed.

It is to be noted that the output section 55 outputs display information in which different region dividing displays are carried out for the same region, and in this case, when the three-dimensionally shown insertion subject 2 is two-dimensionally shown, it is possible to prevent a position corresponding to a depth direction from being unseen or being hard to be seen. Therefore, oversight regions or marking positions can assuredly be recognized.

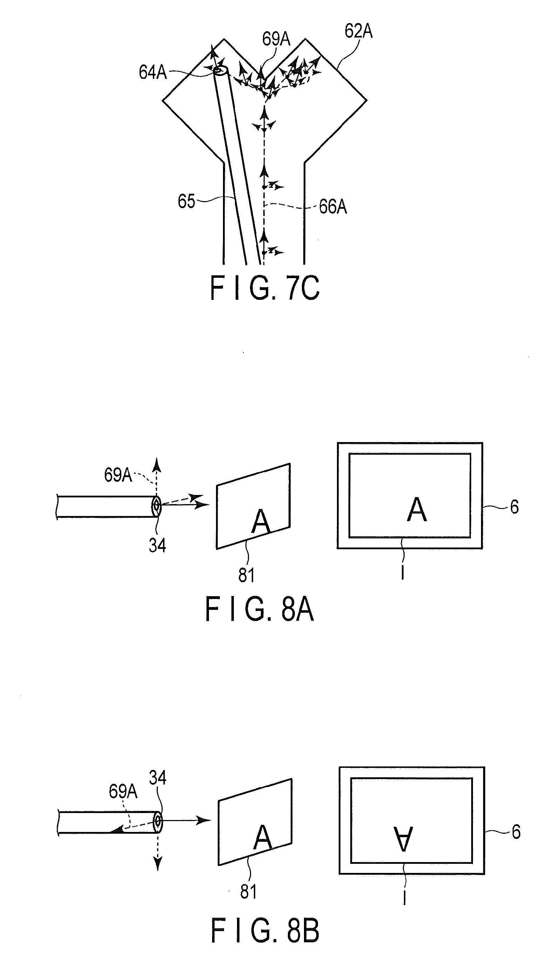

The description has been given as to the example where the image acquisition position is displayed. However, in addition to this position, there may be displayed the position and direction of the inserting section distal end and further, a history of the position and direction of the inserting section distal end. This will be described with reference to FIG. 7A to FIG. 7C and FIG. 8A and FIG. 8B. FIG. 7A to FIG. 7C show content displayed by the display device 6 when the inserting section 31 is inserted into the branched insertion subject 2.

The positional relation calculating section 54 calculates the position and direction of the inserting section distal end and outputs the same to the output section 55 and the storage section 56. It is to be noted that the calculation of the position and direction of the inserting section distal end is similar to the above calculation, and hence description thereof is omitted here.

FIG. 7A shows the inserting section shape schematic display 65 showing the shape of the inserting section 31 at the current time, a current position display 64A showing the current position of a distal position of the inserting section 31 (i.e., the position of the image acquisition opening 34), and a position locus display 66A showing a locus of the position of the image acquisition opening 34, on a two-dimensional view 62A showing the shape of the insertion subject. It is to be noted that the position locus display 66 showing the locus of the image acquisition position is omitted. From the locus display of the distal position, it is possible to recognize a specific position of the insertion subject 2 through which the distal position (i.e., the position of the image acquisition opening 34) passes and a specific position of the insertion subject where the distal position is present at a current time.

When the distal position (i.e., the position of the image acquisition opening 34) is recognized, a specific position of the image acquisition object which is reached is recognized. When the current position is exactly recognized, the observation or treatment to be carried out at the current position or investigation of a path from the current position to a target position can be performed by using this information, without presuming that the current position would be this place. Therefore, it is not necessary to repeat trial and error in reaching the target position, nor is it necessary to confirm whether or not the target position was reached, by various methods including, for example, a method of observing the acquired image. As a result, there is a high possibility that the target position can be reached at one time by taking the path close to the shortest course from the current position to the target position, so that time can be reduced and furthermore, a situation concerning the position can be grasped, which leads to a calmed and assured operation.

In addition to the locus as the history of the distal position (i.e., the position of the image acquisition opening 34) shown in FIG. 7A, a direction in which the inserting section distal end (i.e., the image acquisition opening 34) is directed may be shown. FIG. 7B shows the direction in which the image acquisition opening 34 is directed, as the direction of the inserting section distal end by an arrow 69. In addition to the current position and direction of the image acquisition opening 34, information of directions at several positions on the locus of the image acquisition opening 34 is added by using the arrows 69. From the display of the locus and direction of the distal position, it is possible to recognize the locus of the distal position which is the position information of the image acquisition opening 34 at the inserting section distal end, and a specific direction in which the image acquisition opening is directed while the position of the image acquisition opening changes.

It is to be noted that depending on the optical system for the image acquisition, in the present example, the direction in which the image acquisition opening 34 present at the inserting section distal end is directed is the center of the viewing field and is the middle of the acquired image.

When the distal position and direction are recognized, a position reached and a direction in the image acquisition object are recognized. An observation viewing field direction and the viewing field center are seen from the current position and direction. When the reaching position and direction or the observation viewing field direction and viewing field center are exactly recognized, it is possible to perform the observation or treatment to be carried out in accordance with the current position and direction, or the investigation of the path from the current position to the target position and the shape or operating method of the inserting section 31 during the movement, by use of this information without presuming that the current position and direction would be such the position and direction. In particular, when the direction of the inserting section distal end is recognized, it is possible to investigate an operating method or procedure such as insertion/extraction or bending for the purpose of reaching the target position or direction.

The direction of the inserting section distal end, i.e., the direction in which the image acquisition opening 34 is directed may three-dimensionally be shown to indicate the direction including a posture or rotation of the inserting section distal end.

When the rotation of a coordinate system fixed to the inserting section distal end, i.e., the coordinate system in which the position or posture of the inserting section distal end does not change is defined as "a three-dimensional direction" of the inserting section distal end, FIG. 7C shows the locus of the inserting section distal end in the three-dimensional direction. FIG. 7C shows the direction of the inserting section distal end, i.e., the direction in which the image acquisition opening 34 is directed, by use of arrows 69A of three directions (an x-direction, a y-direction, and a z-direction) to show the three-dimensional direction (the posture).

In addition, information concerning the three-dimensional direction including the rotation may be displayed together with the image acquired by the image acquisition section 32.

When the position and three-dimensional direction of the inserting section distal end are recognized in this manner, for example, the image acquisition direction including the rotation of the inserting section distal end at the image acquisition position is recognized. In addition, an influence of the rotation in the distal end direction can be taken into consideration during the treatment or the like other than the image acquisition.

As to the image acquisition direction including the rotation at the image acquisition position, for example, even in a case where the image acquisition opening 34 is directed in the same direction as shown in FIG. 8A and FIG. 8B, the image acquisition opening 34 to an image acquisition object 81 also rotates when the inserting section 31 rotates round the image acquisition direction. In FIG. 8A and FIG. 8B, the image acquisition opening rotates as much as 180°, and hence the upside and downside are reversed, and in this case, an acquired image I taken by the image acquisition section 32 is also displayed upside-down. It becomes possible to take this influence of the rotation in the image acquisition direction during the observation or treatment into consideration, thus the acquired image can exactly be grasped without mistaking the top and bottom of the image.

### [Second Embodiment]

Next, a second embodiment of the present invention will be described.

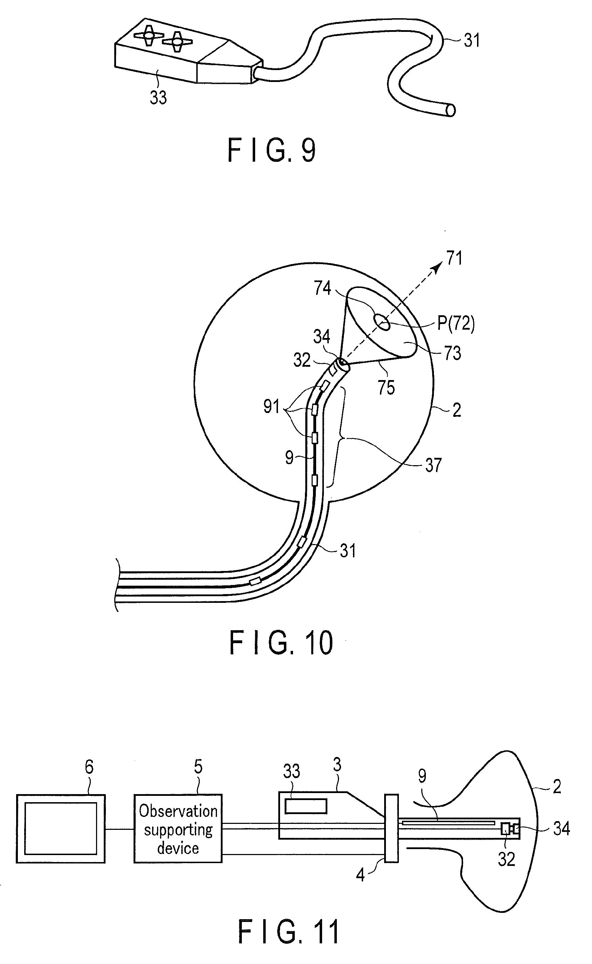

The present second embodiment is an example where an inserting tool 3 is such a soft endoscope device as shown in FIG. 9. This soft endoscope device is different from such a hard endoscope device as described in the above first embodiment in that an inserting section 31 is a flexible tubular member. Furthermore, as shown in FIG. 10, the inserting section 31 has a bending portion 37 in the vicinity of an inserting section distal end, and the bending portion 37 is coupled with an operating lever disposed in an operating section 33 by a wire, though not especially shown in the drawing. In consequence, the operating lever is moved to pull the wire, thereby enabling a bending operation of the bending portion 37.

In this soft endoscope device, unlike such a hard endoscope device as described in the above first embodiment, a shape of the inserting section 31 is not fixed, and the shape of the inserting section 31 changes in accordance with an internal shape of an insertion subject 2 and an operator's bending operation.

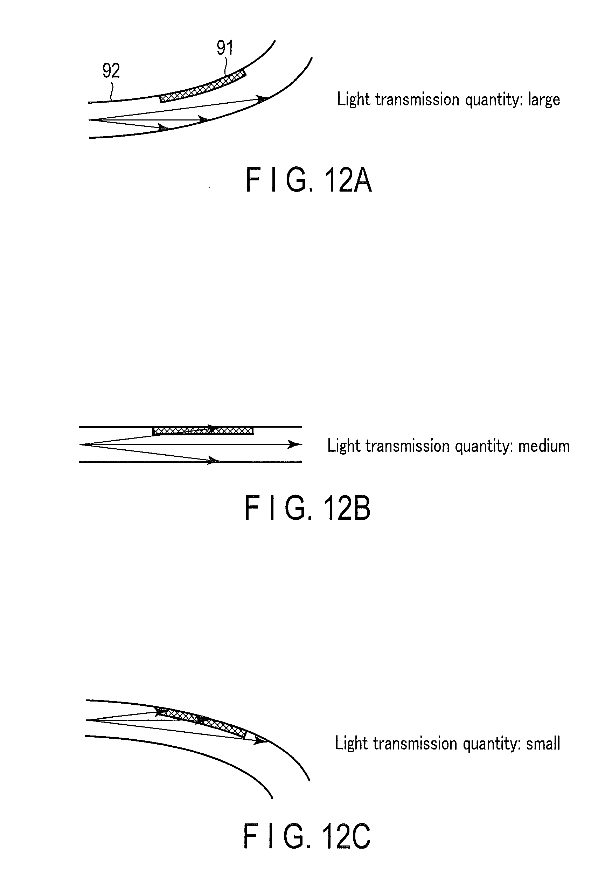

Therefore, in an observation apparatus 1 concerned with the present second embodiment, a fiber shape sensor 9 is disposed in the inserting section 31 as shown in FIG. 11. The fiber shape sensor 9 is constituted of optical fibers, and each optical fiber has a bend detecting portion 91 in one portion thereof. In the bend detecting portion 91, a clad of the optical fiber is removed to expose a core thereof, and a light absorbing material is applied to constitute the bend detecting portion. In the bend detecting portion 91, as shown in FIG. 12A to FIG. 12C, a quantity of light to be absorbed by the bend detecting portion 91 changes in accordance with a bend of the bending portion 37, and hence a quantity of the light to be guided in an optical fiber 92 changes, i.e., a light transmission quantity changes.

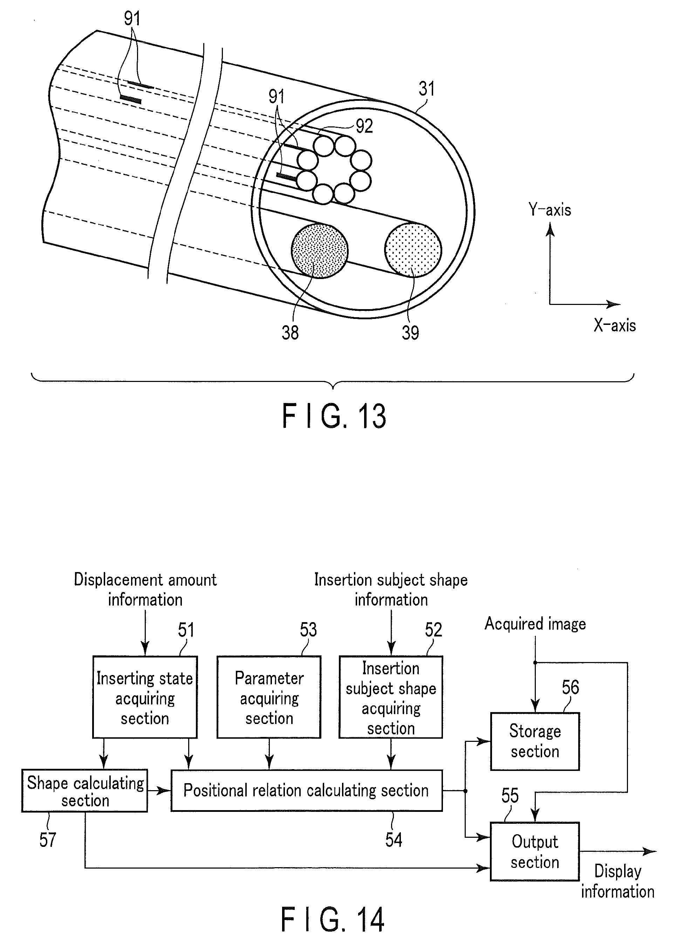

In the fiber shape sensor 9 of this constitution, for the purpose of detecting the bend in an X-axis direction and the bend in a Y-axis direction shown in FIG. 13, two optical fibers 92 are disposed so that the two bend detecting portions 91 directed in the X-axis direction and the Y-axis direction, respectively, form a pair, to detect a bend amount of one region. Furthermore, the optical fibers 92 are disposed so that the pair of bend detecting portions 91 are arranged in a longitudinal direction (an inserting direction) of the inserting section 31. Additionally, light from an unshown light source is guided by each of the optical fibers 92, and the light transmission quantity that changes with the bend amount of each of the optical fibers 92 is detected by an unshown light receiving section. The thus detected light transmission quantity is output as one piece of displacement amount information of the inserting section 31 to an observation supporting device 5.

It is to be noted that the bend detecting portions 91 are preferably disposed not only in the bending portion 37 of the inserting section 31 but also on an operating section side from the bending portion, so that it is possible to also detect a bending state of a portion other than the bending portion 37 of the inserting section 31, which freely bends in accordance with an internal structure of the insertion subject 2 due to flexibility of the inserting section 31.

It is to be noted that as shown in FIG. 13, an illuminating optical fiber 38 and a wiring line 39 for an image acquisition section are also disposed in the inserting section 31. The light from the unshown illuminating light source disposed in the operating section 33 is guided by the illuminating optical fiber 38, and emitted as illuminating light from the inserting section distal end, so that an image acquisition section 32 can acquire image of the inside of the insertion subject 2 that is a dark part.

Additionally, as shown in FIG. 14, the observation supporting device 5 concerned with the present embodiment has a shape calculating section 57 in addition to the above constitution of the first embodiment. An inserting state acquiring section 51 in the present embodiment further acquires a position and a direction of each of the bend detecting portions 91 of the inserting section 31 as inserting state information into the insertion subject 2, on the basis of the light transmission quantity that changes in accordance with the bend amount of each of the optical fibers 92 which is the displacement amount information detected by the fiber shape sensor 9. The shape calculating section 57 calculates the shape of the inserting section 31 on the basis of the position and direction as this inserting state information. In addition, a positional relation calculating section 54 in the present embodiment obtains a positional relation of the inserting section 31 to the insertion subject 2, i.e., an image acquisition position P on the basis of shape information of the inserting section 31 which is obtained by the shape calculating section 57, the inserting state information acquired by the inserting state acquiring section 51, insertion subject shape information acquired by the insertion subject shape acquiring section 52, and parameters acquired by the parameter acquiring section 53. It is to be noted that a movement amount and a moving direction of the inserting section distal end, i.e., a direction (an axial direction) in which the inserting section distal end is directed can be obtained from the shape information of the inserting section 31 and the insertion subject shape information, and hence when the image acquisition position P is calculated, it is not necessary to use any acquired images in the present embodiment.

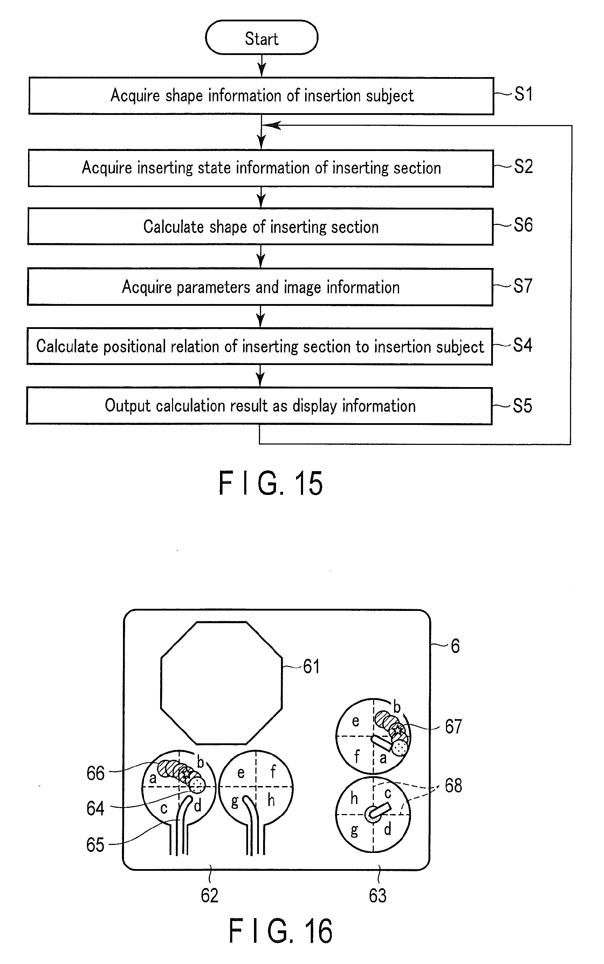

An operation of the observation supporting device 5 of the abovementioned constitution will be described with reference to FIG. 15.

First, the insertion subject shape acquiring section 52 acquires the insertion subject shape information (step S1). Afterward, the inserting state acquiring section 51 acquires the inserting state information of the inserting section 31 into the insertion subject 2 (step S2). Furthermore, the shape calculating section 57 calculates the shape of the inserting section 31 on the basis of the inserting state information acquired by the inserting state acquiring section 51 (step S6). Afterward, the positional relation calculating section 54 acquires the parameters from the parameter acquiring section 53 (step S7), and calculates the positional relation of the inserting section 31 to the insertion subject 2 on the basis of the insertion subject shape information, the inserting state information, the shape information of the inserting section 31 and the parameters (step S4). Furthermore, the calculation result is stored in a storage section 56 and is also output by an output section 55 (step S5), and the process then returns to the step S2 to repeat the above operation of the steps S2, S6, S7, S4 and S5.

By this operation, such a display as shown in FIG. 16 is made in a display device 6 connected to the observation supporting device 5. It is to be noted that in the present embodiment, an inserting section shape schematic display 65 showing the shape of the inserting section 31 corresponds to the shape of the inserting section 31 which is obtained by the shape calculating section 57.

Therefore, even when the shape of the inserting section 31 is not already known, the shape of the inserting section 31 is calculated, so that the operation is performed similarly to the above first embodiment to obtain a similar effect.

The present invention has been described above on the basis of the embodiments, but needless to say, the present invention is not restricted to the abovementioned embodiments and various modifications or applications are possible within the gist of the present invention.

For example, a program of software to realize the function shown in the flowchart of FIG. 5 or FIG. 15 is supplied to a computer, and the computer executes this program to enable realization of the above function.

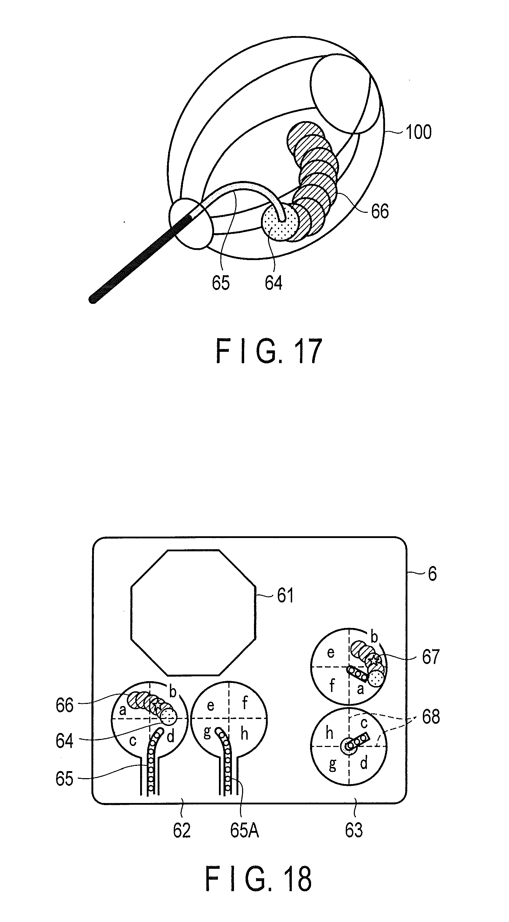

In addition, the display information output by the output section 55, i.e., a display configuration of the display device 6 is not limited to such display as in the two-dimensional views 62 and 63. For example, the display may be made in a three-dimensional view 90 as shown in FIG. 17.

Furthermore, as shown in FIG. 18, a bend detecting section display 65A showing a position of a bend detecting section 100 can be superimposed and displayed on an inserting section shape schematic display 65.

## Claims

1. An observation apparatus **characterized by** comprising:
an inserting section that is to be inserted into an insertion subject;
an image acquisition section that is disposed in the inserting section to acquire image of the insertion subject;
an inserting state acquiring section that acquires inserting state information of the inserting section;
an insertion subject shape acquiring section that acquires shape information of the insertion subject;
a positional relation calculating section that is input the inserting state information and the insertion subject shape information and calculates a positional relation of the inserting section to the insertion subject; and
an output section that outputs the calculation result of the positional relation calculating section as display information.

2. The observation apparatus according to claim 1, **characterized in that** the positional relation calculating section calculates a positional relation of a distal end of the inserting section to the insertion subject.

3. The observation apparatus according to claim 2, **characterized in that**
the positional relation calculating section obtains an image acquisition position of the image acquisition section by use of the positional relation of the inserting section distal end and the shape information of the insertion subject, and
the output section outputs the image acquisition position as the display information.

4. The observation apparatus according to any one of claims 1 to 3, **characterized by** further comprising a storage section that stores at least a part of the calculation result of the positional relation calculating section.

5. The observation apparatus according to any one of claims 1 to 4, **characterized by** further comprising a detecting section that detects an inserting state of the inserting section, and that
the detecting section includes at least one of:
a shape sensor that is mounted in the inserting section and detects a shape of the inserting section; and
an inserting section sensor that is disposed in an insertion port of the inserting section into the insertion subject and detects an insertion amount and a rotation amount when the inserting section passes through the inserting section sensor.

6. The observation apparatus according to any one of claims 1 to 5, **characterized by** further comprising a shape calculating section that calculates a shape of the inserting section on the basis of the inserting state information, and that
the output section outputs the calculation result of the shape calculating section as the display information.

7. The observation apparatus according to claim 2, **characterized in that** the positional relation calculating section further inputs a refractive index of a predetermined material interposed between the inserting section and the insertion subject to obtain the positional relation of the inserting section to the insertion subject.

8. The observation apparatus according to claim 2, **characterized in that** the positional relation calculating section further inputs view angle information of the image acquisition section to obtain the positional relation of the inserting section to the insertion subject.

9. The observation apparatus according to any one of claims 1 to 8, **characterized in that** the output section outputs the display information to display two or more different predetermined states by a distinguishable method, as the result of the positional relation calculating section.

10. The observation apparatus according to claim 4, **characterized in that** the storage section stores an acquired image of the image acquisition section, with the image being associated with the calculation result of the positional relation calculating section.

11. The observation apparatus according to claim 1, **characterized in that** the output section outputs the display information in which region dividing displays are carried out in regions.

12. The observation apparatus according to claim 1, **characterized in that** the output section outputs the display information in which different region dividing displays are carried out for the same region.

13. The observation apparatus according to claim 1, **characterized by** further comprising a display device that displays the display information output from the output section.

14. The observation apparatus according to claim 13, **characterized in that** the output section outputs the display information including information to display the calculation result prior to the current calculation result of the positional relation calculating section as a locus display in the display device.

15. The observation apparatus according to claim 2, **characterized in that**
the positional relation calculating section further obtains a direction of the inserting section distal end to the insertion subject, and
the output section outputs the direction of the distal end of the inserting section as the display information.

16. The observation apparatus according to claim 2, **characterized in that**
the positional relation calculating section further obtains a rotation amount of the inserting section distal end in a direction of the inserting section distal end to the insertion subject, and
the output section outputs the rotation amount in the direction of the inserting section distal end as the display information.

17. An observation supporting device for use in an observation apparatus in which an inserting section is inserted into an insertion subject to acquire image of the inside of the insertion subject, the observation supporting device comprising:
an inserting state acquiring section that acquires inserting state information of the inserting section;
an insertion subject shape acquiring section that acquires shape information of the insertion subject;
a positional relation calculating section that is input the inserting state information and the insertion subject shape information and calculates a positional relation of the inserting section to the insertion subject; and
an output section that outputs the calculation result of the positional relation calculating section as display information.

18. An observation supporting method for use in an observation apparatus in which an inserting section is inserted into an insertion subject to acquire image of the inside of the insertion subject, the method comprising:
an inserting state acquiring step of acquiring inserting state information of the inserting section;
an insertion subject shape acquiring step of acquiring shape information of the insertion subject;
a positional relation calculating step of being input the inserting state information and the insertion subject shape information, and calculating a positional relation of the inserting section to the insertion subject; and
an output step of outputting the calculation result of the positional relation calculating step as display information.

19. A program which allows a computer to execute:
an inserting state acquiring procedure of acquiring inserting state information of an inserting section in an observation apparatus in which the inserting section is inserted into an insertion subject to acquires image of the inside of the insertion subject;
an insertion subject shape acquiring procedure of acquiring shape information of the insertion subject;
a positional relation calculating procedure of being input the inserting state information and the insertion subject shape information, and calculating a positional relation of the inserting section to the insertion subject; and
an output procedure of outputting the calculation result of the positional relation calculating procedure as display information.
